# EUROPEAN PATENT SPECIFICATION

(11) **EP 4 129 167 B1**
(45) Date of publication and mention of the grant of the patent: **27.08.2025**
(21) Application number: 21792900.9
(22) Date of filing: 30.03.2021
(51) Int. Cl.: A61B 5/053

(54) **BODY COMPOSITION DETECTION DEVICE**
VORRICHTUNG ZUR ERKENNUNG DER KÖRPERZUSAMMENSETZUNG
DISPOSITIF DE DÉTECTION DE COMPOSITION CORPORELLE

(30) Priority: 23.04.2020 CN 202010326694
(43) Date of publication of application: 08.02.2023
(73) Proprietor: Huawei Technologies Co., Ltd., Shenzhen, Guangdong 518129 (CN)
(72) Inventor: ZHAO, Shuai, Shenzhen, Guangdong 518129 (CN); YANG, Bin, Shenzhen, Guangdong 518129 (CN); REN, Huichao, Shenzhen, Guangdong 518129 (CN); LI, Yue, Shenzhen, Guangdong 518129 (CN)
(74) Representative: Pfenning, Meinig & Partner mbB
(86) International application number: PCT/CN2021/083893
(87) International publication number: WO 2021/213143

(56) References cited:
- EP-A1- 0 749 718
- EP-A1- 1 512 371
- WO-A1-2013/018295
- CN-A- 106 061 377
- CN-A- 107 063 418
- CN-A- 107 157 481
- CN-A- 107 913 063
- CN-A- 108 451 529
- CN-A- 109 069 055
- CN-U- 206 459 735
- CN-Y- 2 529 252
- CN-Y- 2 935 300
- US-A- 5 475 933
- US-A1- 2016 256 070
- US-A1- 2017 105 646
- US-A1- 2018 271 446
- US-A1- 2019 079 038

## Description

### TECHNICAL FIELD

This application relates to the field of electronic device technologies, and in particular, to a body composition detection method and device.

### BACKGROUND

A body composition is content of fat, muscle, protein, minerals, and other components in a human body. The body composition can be used to assess development, maturity, and aging statuses of the human body, and has great significance in monitoring child development, providing guidance for bodybuilding and slimming, and healthcare.

Common body composition detection methods include dual energy X-ray absorptiometry (dual energy X-ray absorptiometry, DEXA) and bioelectrical impedance analysis (bioelectrical impedance analysis, BIA). In the DEXA, absorption and attenuation of X-rays at different energy levels passing through bones and soft tissues of a human body are measured to obtain a body composition. This method is highly accurate, but incurs radiation and high costs. When different weak currents pass through a human body, fat, muscle, and other components in the human body have different electrical conductivity, and generate different body impedance. Therefore, in the BIA, content of various body components is calculated by using measured impedance in combination with information about a person, for example, a sex, an age, a height, and a weight. The BIA measurement method is simple and easy to use, and is a main measurement method for a professional body fat scale and a household body fat scale.

Accuracy of BIA-based body composition detection depends on an impedance measurement result, and accuracy of body impedance detection is greatly affected by humidity on a skin surface. When feet and hands of a user are sweaty, or when a user performs body composition measurement immediately after exercise or a shower with an expectation of achieving a fat reduction effect, because surfaces of the feet and hands contain sweat or moisture, a large impedance measurement error occurs, and consequently, a body composition detection result is inaccurate. Further technological background can be found in the following publications US 2019/079038A1 which concerns a moisture sensitive sheet that includes a stretchable circuit board, US 2018/271446 A1 which concerns a smart toilet with a function of detecting the body composition, US 2016/256070A1 which concerns a moisture sensor that includes a pair of electrode plates separated by a moisture absorbent material that forms the dielectric of a capacitive sensor, WO 2013/018295A1 which concerns a body moisture meter and US 2017/105646 A1 which concerns a physiological sensor system to be worn on a subject's skin to measure, process or store one or more physiological parameters.

### SUMMARY

Embodiments of this application provide a body composition detection method and device, to improve accuracy for detecting a body composition by using body impedance. The invention as claimed is defined in independent claim 1.

A first aspect of this application provides a body composition detection device, including a housing, a support layer, and at least one detector. The detector includes an impedance measurement assembly and a dehumidification assembly. The support layer includes a processor. Both the impedance measurement assembly and the dehumidification assembly are electrically connected to the processor. The impedance measurement assembly, the dehumidification assembly, and the processor are all disposed inside the housing. The impedance measurement assembly is embedded on an upper surface of the housing. An upper surface of the impedance measurement assembly is flush with or higher than the upper surface of the housing. The upper surface of the impedance measurement assembly is in contact with a human body during measurement. The dehumidification assembly is added in the detection device, so that the dehumidification assembly dehumidifies a body surface in contact with the impedance measurement assembly before the impedance measurement assembly measures body impedance. After moisture on the body surface is removed, the body impedance measurement assembly measures the body impedance. In this way, accuracy of the body impedance measurement is improved, and further, accuracy for detecting a body composition by using the body impedance can be improved.

In the first aspect, the detector further includes a humidity measurement assembly. The humidity measurement assembly is electrically connected to the processor. The humidity measurement assembly is embedded in the impedance measurement assembly. An upper surface of the humidity measurement assembly is in contact with a human body during measurement. Correspondingly, when a humidity value measured by the humidity measurement assembly does not meet a measurement condition requirement of the impedance measurement assembly, the dehumidification assembly performs dehumidification; or when the humidity value measured by the humidity measurement assembly meets the measurement condition requirement of the impedance measurement assembly, dehumidification does not need to be performed, and the impedance measurement assembly measures the body impedance, so that the detection device can perform dehumidification based on an actual situation, thereby providing a user with better experience.

Optionally, the upper surface of the humidity measurement assembly and the upper surface of the impedance measurement assembly are located on one plane, to ensure comfort during measurement by a user.

In an example manner, the dehumidification assembly is an electric heating element, and the impedance measurement assembly, the housing, the electric heating element, and the support layer are stacked from top to bottom. A through hole is provided on each of the impedance measurement assembly, the housing, and the electric heating element. The humidity measurement assembly is mounted in the through hole, and a lower end of the humidity measurement assembly is electrically connected to the support layer.

Optionally, the through hole is provided at centers of the impedance measurement assembly and the electric heating element.

In another example manner, the dehumidification assembly is a fan, and the impedance measurement assembly, the housing, the fan, and the support layer are stacked from top to bottom. A through hole is provided on each of the impedance measurement assembly, the housing, and the fan. The humidity measurement assembly is mounted in the through hole, and a lower end of the humidity measurement assembly is electrically connected to the processor. A plurality of air vents are provided at corresponding positions on the impedance measurement assembly and the housing, and air blown out of the fan reaches the body surface through the plurality of air vents.

Optionally, the through hole is provided at centers of the impedance measurement assembly, the housing, and a rotating shaft of the fan.

In another example manner, the dehumidification assembly is a fan, and the impedance measurement assembly, the housing, the fan, and the support layer are stacked from top to bottom. A through hole is provided on each of the impedance measurement assembly and the housing. The humidity measurement assembly is mounted in the through hole, and an electrode of the humidity measurement assembly and an electrode of the impedance measurement assembly are connected to the processor through a wire duct on an upper surface of the fan. A plurality of air vents are provided at corresponding positions on the impedance measurement assembly and the housing, and air blown out of the fan reaches the body surface through the plurality of air vents.

Optionally, the through hole is provided at centers of the impedance measurement assembly and the housing.

In still another example manner, the dehumidification assembly is a drying ring, the impedance measurement assembly is made of a super-hydrophobic conductive material, the impedance measurement assembly has an arc-shaped convex structure, and the drying ring is arranged around the impedance measurement assembly. An upper surface of the drying ring is lower than or flush with an outer edge of the impedance measurement assembly, so that a slope can be formed. In this way, water droplets on the body surface can fall from the top of the impedance measurement assembly onto the drying ring, and the drying ring absorbs moisture.

Optionally, the impedance measurement assembly is an impedance measurement electrode, and the impedance measurement electrode is formed by coating the upper surface of the housing.

Optionally, the impedance measurement assembly is disposed in a groove of the housing.

In an example manner, the body composition detection device further includes a pressure sensor, configured to measure a body weight. It can be understood that measurement needs to be performed in combination with the body weight when a body composition is measured.

Optionally, the pressure sensor is disposed below the support layer.

Optionally, the upper surface of the humidity measurement assembly and the upper surface of the impedance measurement assembly are located on one plane.

In an example manner, the body composition detection device includes four detectors, and the four detectors are symmetrically distributed around a center of the detection device.

In an example manner, the processor is further configured to: when the humidity value measured by the humidity measurement assembly does not meet the measurement condition requirement of the impedance measurement assembly, output first prompt information, where the first prompt information is used to prompt whether to perform dehumidification; and when a dehumidification confirmation instruction entered by the user based on the first prompt information is received, perform dehumidification by using the dehumidification assembly.

In an example manner, the processor is further configured to: when the humidity value measured by the humidity measurement assembly meets the measurement condition requirement, control the dehumidification assembly to stop dehumidification; or when a preset dehumidification time expires, control the dehumidification assembly to stop dehumidification.

A non-claimed second aspect of this application provides a body composition detection method, applied to a body composition detection device. The body composition detection device includes a dehumidification assembly, and the dehumidification assembly is configured to dehumidify a body surface under measurement. After dehumidification is completed, body impedance is measured. The detection device may process a measured body impedance value to obtain a body composition, and output the body composition; or the detection device sends a measured body impedance value to a terminal device, and the terminal device processes the body impedance value to obtain a body composition, and outputs the body composition. In the method, the body surface is dehumidified before the body impedance is measured, and the body impedance is measured only after moisture on the body surface is removed. In this way, accuracy of the body impedance measurement is improved, and further, accuracy for detecting the body composition by using the body impedance can be improved.

In an example manner, before the dehumidification assembly dehumidifies the body surface that is in contact, humidity on the body surface is measured.

Optionally, after measuring the humidity on the body surface, the body composition detection device determines whether a measured humidity value of the body surface meets a measurement condition requirement. When the humidity value of the body surface does not meet the measurement condition requirement, the dehumidification assembly dehumidifies the body surface; or when the humidity value of the body surface meets the measurement condition requirement, the body impedance is directly measured. The humidity on the body surface is detected, and whether dehumidification is to be performed is determined based on the humidity on the body surface, thereby improving user experience.

Alternatively, after measuring the humidity on the body surface, the body composition detection device sends the humidity value of the body surface to the terminal device, so that the terminal device determines whether dehumidification is to be performed.

In an example manner, in a process of dehumidifying the body surface by the dehumidification assembly, the humidity on the body surface is detected, and when the humidity on the body surface meets the measurement condition requirement, it is determined that dehumidification is completed, and the body impedance measurement starts.

In another example manner, when a preset dehumidification time expires, it is determined that dehumidification is completed, and the body impedance measurement starts. The dehumidification time may be preset by a system, or may be sent by the terminal device to the body composition detection device.

Optionally, when the humidity value of the body surface does not meet the measurement condition requirement, the detection device outputs first prompt information, where the first prompt information is used to prompt whether to perform dehumidification. When a dehumidification confirmation instruction entered by a user based on the first prompt information is received, the dehumidification assembly dehumidifies the body surface. Interaction is performed with the user, so that the user determines whether dehumidification is to be performed. In this way, requirements of different users can be met, and the user is provided with better experience.

Optionally, when a dehumidification denial instruction entered by a user based on the first prompt information is received, second prompt information is output, where the second prompt information is used to prompt the user to perform detection after removing moisture.

In an example manner, after dehumidification is completed and before the body impedance is measured, third prompt information is further output, where the third prompt information is used to prompt a start of the body impedance measurement. The third prompt information is output, so that the user can learn of a current measurement progress in a timely manner.

In an example manner, the dehumidification assembly performs dehumidification in one or more of the following manners: heating, blowing, or moisture absorption.

In an example manner, before the dehumidification assembly dehumidifies the body surface that is in contact, the dehumidification assembly receives first indication information sent by the terminal device, where the first indication information is used to indicate to perform dehumidification before the body impedance is measured; and in response to the first indication information, the dehumidification assembly dehumidifies the body surface.

Further, the body composition detection device further receives a dehumidification time sent by the terminal device, and when the dehumidification time expires, determines that dehumidification is completed. Optionally, the terminal device may indicate the dehumidification time while indicating the body composition detection device to perform dehumidification.

In an example manner, after dehumidification is completed and before the body impedance is measured, the body composition detection device receives second indication information sent by the terminal device, where the second indication information is used to indicate to stop dehumidification and start body impedance measurement. The body composition detection device starts to measure the body impedance based on the second indication information.

In an example manner, after obtaining body composition information through processing, the body composition detection device sends the body composition information to the terminal device, and the terminal device displays the body composition information.

A non-claimed third aspect of this application provides a body composition method, including: A terminal device sends first indication information to a body composition detection device, where the first indication information is used to indicate the body composition detection device to perform dehumidification before measuring body impedance. The terminal device receives a body impedance value sent by the detection device, processes the body impedance value to obtain a body composition, and displays the body composition. The terminal device indicates the body composition detection device to dehumidify a body surface before measuring the body impedance, and measure the body impedance only after moisture on the body surface is removed. In this way, accuracy of the body impedance measurement is improved, and further, accuracy for detecting the body composition by using the body impedance can be improved.

In an example manner, before the terminal device sends the first indication information to the body composition detection device, the terminal device displays first prompt information, where the first prompt information is used to prompt whether to perform dehumidification, and when a dehumidification confirmation instruction entered by a user based on the first prompt information is received, the terminal device sends the first indication information to the body composition detection device.

In an example manner, before displaying the first prompt information, the terminal device receives a humidity value of the body surface that is sent by the detection device; and when the humidity value of the body surface does not meet a measurement condition requirement, the terminal device displays the first prompt information; or when the humidity value of the body surface meets the measurement condition requirement, the terminal device indicates the detection device to start body impedance measurement.

Optionally, when the humidity value of the body surface meets the measurement condition requirement, the terminal device sends second indication information to the detection device, to indicate to stop dehumidification and start body impedance measurement.

In an example manner, the terminal device sends a dehumidification time to the detection device, where the dehumidification time may be flexibly set by the user.

In an example manner, when a dehumidification denial instruction entered by a user based on the first prompt information is received, the terminal device displays second prompt information, where the second prompt information is used to prompt the user to perform detection after removing moisture.

In an example manner, after the body composition detection device completes dehumidification and before the body composition detection device measures the body impedance, the terminal device displays third prompt information, where the third prompt information is used to prompt a start of body impedance measurement.

A non-claimed fourth aspect of this application provides a computer-readable storage medium. The computer-readable storage medium stores computer-executable instructions. When the computer-executable instructions are executed by a processor, the computer-executable instructions are used to implement the body composition detection method according to the second aspect, the third aspect, or each of the example manners.

A non-claimed fifth aspect of this application provides a program. When the program is executed by a processor, the program is used to perform the body composition detection method according to the second aspect, the third aspect, or each of the example manners.

A non-claimed sixth aspect of this application provides a chip, including a processing module and a communications interface. The processing module can perform the body composition detection method according to the second aspect, the third aspect, or each of the example manners.

Embodiments of this application provide a body composition detection method and device. The body composition detection device includes a housing, a support layer, and at least one detector. The detector includes an impedance measurement assembly and a dehumidification assembly. The support layer includes a processor. Both the impedance measurement assembly and the dehumidification assembly are electrically connected to the processor. The impedance measurement assembly, the dehumidification assembly, and the processor are all disposed inside the housing. The impedance measurement assembly is embedded on an upper surface of the housing. An upper surface of the impedance measurement assembly is flush with or higher than the upper surface of the housing. The upper surface of the impedance measurement assembly is in contact with a human body during measurement. Because moisture on a body surface greatly affects body impedance measurement, the dehumidification assembly removes the moisture on the body surface before body impedance is measured, and measurement is performed after the body surface is dried. This can improve accuracy of the body impedance measurement, and further can improve accuracy for detecting a body composition by using the body impedance.

### BRIEF DESCRIPTION OF DRAWINGS

FIG. 1 is a schematic diagram of an external structure of a body composition detection device;
FIG. 2 is a schematic diagram of a body composition measurement scenario;
FIG. 3 is a schematic diagram of a body composition measurement process;
FIG. 4 is a schematic diagram of an interface for setting a dehumidification time;
FIG. 5 is a schematic diagram of a display interface of a detection device;
FIG. 6 is a schematic diagram of a dehumidification interface of a terminal device;
FIG. 7 is a schematic diagram of another external structure of a body composition detection device;
FIG. 8 is a schematic diagram of still another external structure of a body composition detection device;
FIG. 9 is a schematic diagram of an external structure of a hand detection device;
FIG. 10 is a schematic diagram of another external structure of a hand detection device;
FIG. 11 is a schematic diagram of still another external structure of a hand detection device;
FIG. 12 is a schematic exploded view of a detector of a body composition detection device that performs dehumidification based on heating;
FIG. 13 is a schematic diagram of an appearance of the detector of the body composition detection device shown in FIG. 12;
FIG. 14 is a schematic exploded view of a detector of a body composition detection device that performs dehumidification based on blowing;
FIG. 15 is a schematic diagram of an appearance of the detector of the body composition detection device shown in FIG. 14;
FIG. 16 is a schematic exploded view of a detector of a body composition detection device that performs dehumidification based on moisture absorption;
FIG. 17 is a side view of an appearance of the detector of the body composition detection device shown in FIG. 16;
FIG. 18 is a flowchart of a body composition detection method according to Embodiment 4 of this application;
FIG. 19 is a flowchart of a body composition detection method according to Embodiment 5 of this application; and
FIG. 20 is a schematic diagram of a structure of a body composition detection device according to Embodiment 6 of this application.

### DESCRIPTION OF EMBODIMENTS

General embodiments of this application provide a body composition detection device and method, wherein embodiments according to the claimed invention are defined in the claims. The body composition detection device detects a body composition based on BIA. BIA-based body composition detection has a simple measurement method and is easy to use, and therefore has been widely used. Common body composition detection devices include a professional body fat scale and a household body fat scale.

The body composition detection device may be a foot measurement device, or may be a hand measurement device, or may include a foot measurement device and a hand measurement device. Accuracy of the BIA-based body composition detection depends on an impedance measurement result, and accuracy of body impedance detection is greatly affected by humidity on a skin surface. When feet or hands of a user are sweaty or have moisture, for example, the hands and the feet of the user are sweaty after the user takes exercise, or the hands or the feet of the user have moisture after the user takes a shower or washes up, a measurement result obtained by measuring body impedance has a large error.

In a conventional technology, "suggestion" and "reminder" information is usually added to a manual of a body composition detection device, for example, "avoiding usage after exercise or a shower", to prompt a user to avoid using the body composition detection device when feet or hands are sweaty or have moisture.

That a user uses a body composition detection device immediately after exercise or a shower is a main application scenario. Usually, a user performs body composition measurement immediately after exercise or a shower with an expectation of achieving a fat reduction effect. Therefore, how to avoid body composition detection or improve accuracy of body composition detection when feet or hands of a user are sweaty is an issue that urgently needs to be addressed.

To address the issue in the conventional technology, embodiments of this application provide a body composition detection device. The body composition detection device has a dehumidification function, and can remove moisture on a contact part (a hand or a foot) of a human body before measuring body impedance, thereby ensuring that a body impedance measurement result is accurate, and improving accuracy of body composition detection.

The body composition detection device may be used in combination with a terminal device, for example, a mobile phone, a tablet computer, a wearable device, or a personal computer. The terminal device communicates with and interacts with the body composition detection device by using an installed body composition detection app. For example, the terminal device displays a body composition detection result. A user may further set the body composition detection device by using the terminal device.

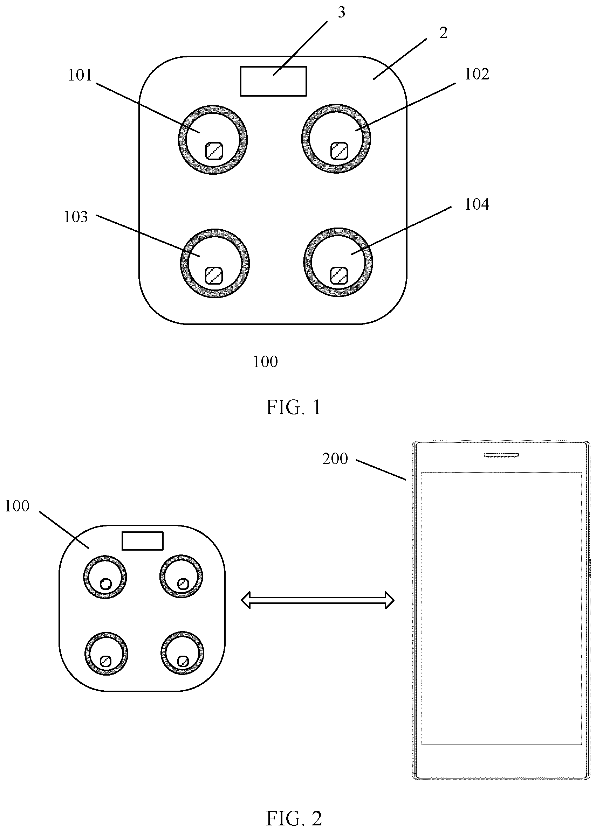

FIG. 1 is a schematic diagram of an external structure of a body composition detection device. As shown in FIG. 1, the body composition detection device 100 includes a housing 2, a support layer (located inside the housing and not shown in the figure), and four detectors: a detector 101, a detector 102, a detector 103, and a detector 104. The four detectors are symmetrically distributed around a center of the detection device 100.

It can be understood that FIG. 1 is merely an example, and the detection device 100 may alternatively include more or fewer detectors.

Each detector includes an impedance measurement assembly and a dehumidification assembly. The support layer includes a processor. Both the impedance measurement assembly and the dehumidification assembly are electrically connected to the processor. The impedance measurement assembly, the dehumidification assembly, and the support layer are all disposed inside the housing. An upper surface of the impedance measurement assembly is flush with or higher than an upper surface of the housing. The upper surface of the impedance measurement assembly is in contact with a human body during measurement.

The dehumidification assembly is configured to dehumidify a body surface in contact with the impedance measurement assembly. The impedance measurement assembly is configured to measure body impedance after the dehumidification assembly completes dehumidification. The processor is configured to process the body impedance measured by the impedance measurement assembly to obtain a body composition, and output the body composition. Alternatively, the processor sends the body impedance measured by the impedance measurement assembly to a terminal device (for example, a mobile phone) that controls the detection device 100, and the terminal device processes the body impedance to obtain a body composition, and outputs the body composition.

When the detection device 100 shown in FIG. 1 is used for measurement, both feet of a human body step on the detection device 100, and the detection device 100 is woken up by a body weight, and can start measurement. When a user measures only a body weight, the user may step on the detection device 100 while wearing shoes and socks, and stepping positions of feet of the user are not limited. When a user needs to measure a body composition, the user needs to step on the detection device with bare feet, and cannot wear shoes or socks, and stepping positions of the feet of the user need to be fixed positions. For example, a left foot of a human body steps on the detector 101 and the detector 103, and a right foot of the human body steps on the detector 102 and the detector 104. Otherwise, measurement cannot be performed, or a measurement result may be inaccurate.

When the detection device 100 is used to measure a body composition, there are the following two scenarios. Scenario 1: The detection device 100 independently completes body composition measurement and outputs a body composition. Scenario 2: As shown in FIG. 2, the detection device 100 cooperates with a connected terminal device 200 to perform measurement, and the terminal device 200 outputs a body composition.

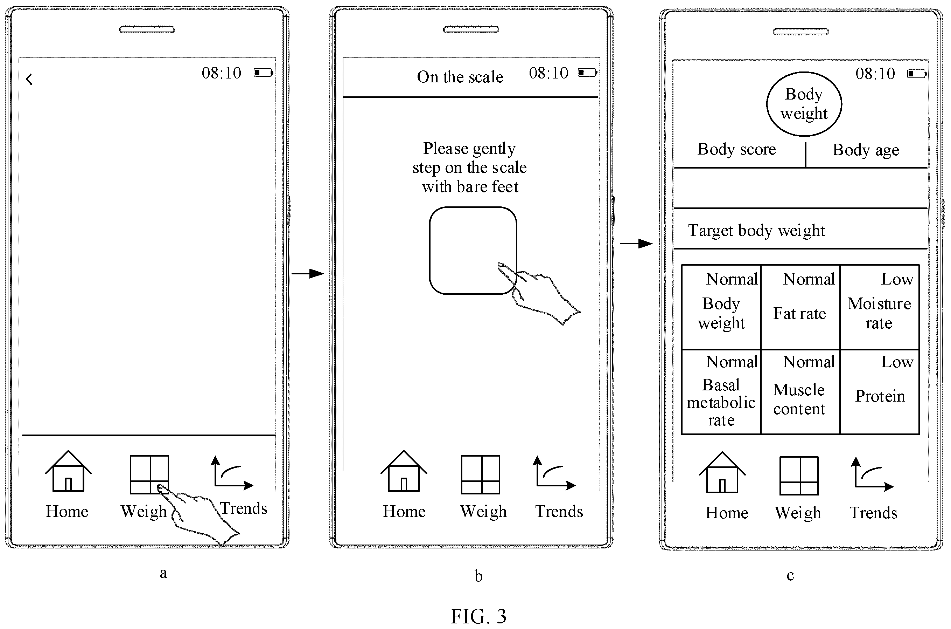

The scenario 2 is used as an example. Before measurement, a user first starts a body composition measurement app or a body fat scale app installed on the terminal device 200. FIG. 3 is a schematic diagram of a body composition measurement process. As shown in FIG. 3, after a user taps the body fat scale app, an interface shown in FIG. 3(a) is displayed by default, and the interface may display user information and a previous body composition measurement result. A menu bar including Home, Weigh, and Trends is further displayed at the bottom of the interface. "Home" is the interface shown in FIG. 3(a). After the user taps "Weigh", an interface shown in FIG. 3(b) is displayed. "Trends" can display change trends of a body weight, fat, muscle, or the like of the user over a period of time in curves.

The interface shown in FIG. 3(b) displays a thumbnail and usage tip information of an electronic scale. For example, the usage tip information is "please gently step on the scale with bare feet". After stepping on the detection device 100, the user taps the thumbnail of the electronic scale to trigger measurement. After measuring body impedance, the detection device 100 sends the body impedance to the terminal device 200. The terminal device 200 processes the body impedance to obtain a body composition, and displays a body composition measurement result on an interface shown in FIG. 3(c). As shown in FIG. 3(c), the body composition measurement result includes a body score, a body age, a current body weight, a fat rate, a moisture rate, a basal metabolic rate, protein content, muscle content, and the like. Optionally, a target body weight and a difference between the current body weight and the target body weight are further displayed.

Optionally, in the scenario 2, after measuring body impedance, the detection device 100 may alternatively process the body impedance to obtain a body composition, and send the body composition to the terminal device 200 for displaying.

In the scenario 1, because a display of the detection device 100 is small, a body composition measurement result may not be completely displayed on one screen. In this case, measurement results of body components may be sequentially displayed in a scrolling display manner.

Optionally, a current- and voltage-based impedance measurement manner may be used for the detector. When the current- and voltage-based impedance measurement manner is used, a current loop needs to be formed. In the detection device 100 shown in FIG. 1, electrodes of two impedance measurement assemblies of the detector 101 and the detector 102 form a current loop, and a current flows into a human body from the detector 101, passes through the human body, and flows out of the human body from the detector 102. Electrodes of two impedance measurement assemblies of the detector 103 and the detector 104 are configured to measure a voltage. The impedance measurement assembly calculates body impedance based on the current and the measured voltage.

In this embodiment, the dehumidification assembly may or may not be in contact with a body surface in a dehumidification process. Whether the dehumidification assembly is in contact with a human body in a dehumidification process is related to a structure or a dehumidification manner of the dehumidification assembly. The dehumidification assembly may perform dehumidification in one or more manners, such as heating, blowing, or moisture absorption. This is not limited in this embodiment.

The support layer is configured to support or mount the impedance measurement assembly and the dehumidification assembly. The processor has a control function for controlling each component in the detector, a data processing function, a user information exchange function, and a function of data communication with the terminal device.

The processor may be implemented by one or more application-specific integrated circuits (Application Specific Integrated Circuit, ASIC), digital signal processors (Digital Signal Processing, DSP), digital signal processing devices (Digital Signal Processing Device, DSPD), programmable logic devices (programmable logic device, PLD), field programmable gate arrays (Field Programmable Gate Array, FPGA), controllers, micro-controllers, microprocessors, or other electronic elements.

Optionally, the support layer further includes a memory, a connection wire, a communication module, and the like. The connection wire is configured to connect to another component in the detector, and implement connection between detectors. The communication module is configured to implement communication between the detection device 100 and the terminal device. The memory is configured to store computer-executable instructions. The processor executes the computer-executable instructions stored in the memory.

Optionally, after the detection device 100 is started, the dehumidification assembly may automatically trigger dehumidification on a body surface that is in contact. After the dehumidification ends, the impedance measurement assembly starts to measure body impedance. The dehumidification assembly may perform dehumidification based on a preset dehumidification time. After the dehumidification time expires, the dehumidification ends. By default, it is considered that the body surface is dry and body impedance measurement can be performed. The dehumidification time may be a system default value, or may be flexibly set by a user, where the user sets the dehumidification time by using the body composition measurement app or the body fat scale app on the terminal device.

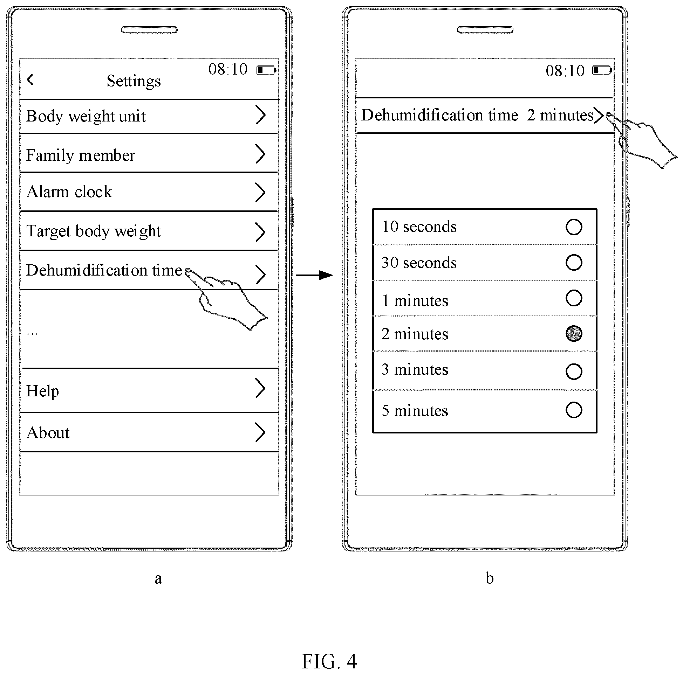

FIG. 4 is a schematic diagram of an interface for setting a dehumidification time. As shown in FIG. 4, the user opens a settings interface shown in FIG. 4(a) in the body fat scale app. The settings interface includes the following settings items: a body weight unit, a family member, an alarm clock, a target body weight, help, about, and the like. The user may set a unit of a body weight to kg (kg), pound (lb), or the like by using the "body weight unit" settings item. The user may add a plurality of members by using the "family member" settings item, to measure and manage body fat and body compositions of the members. The user may set a body composition measurement time by using the "alarm clock" settings, and the user may set, by using the "target body weight" settings item, a body weight value that the user wants to reach. The user may set a dehumidification time by using the "dehumidification time" settings item.

After the user taps the "dehumidification time" settings item in FIG. 4(a), the page jumps to a dehumidification time settings page shown in FIG. 4(b), where a current dehumidification time is displayed at the top of the page. After the user taps the current dehumidification time, a settings dialog box is displayed, and the settings dialog box includes a plurality of time options: 10 seconds, 30 seconds, 1 minute, 2 minutes, 3 minutes, or 5 minutes. The user taps a selection button after a time option to complete the settings of the dehumidification time.

Optionally, after the detection device 100 is started, the dehumidification assembly may alternatively perform dehumidification under triggering by another trigger condition.

In an optional embodiment, at least one detector of the detection device 100 may further include a humidity measurement assembly. The humidity measurement assembly is electrically connected to the processor. The humidity measurement assembly is embedded in the impedance measurement assembly. An upper surface of the humidity measurement assembly is in contact with a human body during measurement.

The humidity measurement assembly is configured to measure humidity on a body surface, and send a measured humidity value to the processor. The processor is configured to: when the humidity value measured by the humidity measurement assembly does not meet a measurement condition requirement of the impedance measurement assembly, control the dehumidification assembly to perform dehumidification. Optionally, the processor may alternatively send the humidity value to the terminal device 200, and the terminal device 200 determines, based on the humidity value, whether dehumidification is to be performed. If dehumidification needs to be performed, the terminal device 200 may send first indication information to the detection device 100, where the first indication information is used to instruct the detection device 100 to perform dehumidification.

Optionally, in this embodiment of this application, one humidity measurement assembly may be disposed in each detector, or humidity measurement assemblies may be disposed in only some detectors. For example, the detection device includes four detectors, and a humidity measurement assembly may be disposed in only one of the detectors, or humidity measurement assemblies may be disposed in two of the detectors, or a humidity measurement assembly may be disposed in each of the four detectors.

To measure humidity on a body surface, the humidity measurement assembly needs to be in contact with the body surface. Because the upper surface of the impedance measurement assembly also needs to be in contact with a human body during measurement, optionally, the humidity measurement assembly may be embedded in the impedance measurement assembly, and an upper surface of the humidity measurement assembly and the upper surface of the impedance measurement assembly are located on one plane. This can ensure that both the upper surface of the humidity measurement assembly and the upper surface of the impedance measurement assembly are in contact with the body surface, while ensuring comfort during measurement.

For example, the measurement condition requirement is as follows: Dehumidification needs to be performed when a humidity value is greater than or equal to a preset humidity threshold. For example, after the detection device 100 is woken up, the humidity measurement assembly starts to measure humidity on a body surface; and when a measured humidity value is greater than or equal to the preset humidity threshold, the processor determines that the body surface is to be dehumidified, and controls the dehumidification assembly to start dehumidification; or when a measured humidity value is less than the preset humidity threshold, the processor determines that the body surface is not to be dehumidified. Optionally, the terminal device 200 may determine, based on a measurement condition requirement that is the same as that of the detection device 100, whether dehumidification is to be performed. Certainly, the terminal device may alternatively determine, based on another condition, whether dehumidification is to be performed. This is not limited in this embodiment of this application.

In an optional implementation, after determining (including determining by the detection device and determining by the terminal device) that the body surface is to be dehumidified, the processor may control the dehumidification assembly to perform dehumidification based on a preset dehumidification time, and end dehumidification after the dehumidification time expires. The dehumidification time may be a system default value, or may be flexibly set by a user on the terminal device and dynamically sent to the detection device.

In another optional implementation, the processor may determine a dehumidification time based on a measured humidity value; or the terminal device determines a dehumidification time based on a measured dehumidification value, and sends the dehumidification time to the detection device; and the processor of the detection device controls the dehumidification assembly to perform dehumidification based on the dehumidification time, and end dehumidification after the dehumidification time expires.

In still another optional implementation, in a dehumidification process of the dehumidification assembly, the humidity measurement assembly continuously measures humidity on the body surface, and when a measured humidity value meets the measurement condition requirement of the impedance measurement assembly, the dehumidification assembly is controlled to end the dehumidification operation. For example, when the measured humidity value is less than the preset humidity threshold, dehumidification is stopped.

Optionally, the humidity measurement assembly includes one or more humidity sensors. The humidity sensor may be a micro-electro-mechanical system (Micro-Electro-Mechanical System, MEMS) touch sensor.

When the detection device 100 includes a foot measurement device, the foot measurement device may be woken up (or started) by a body weight. When measurement needs to be performed, a user stands on the detection device 100. When the detection device 100 detects a weight and the detected weight is greater than a specified threshold, the detection device 100 is started, and the impedance measurement assembly, the dehumidification assembly, and the processor can start to operate. This embodiment is merely an example for description. The detection device 100 may alternatively be started in another manner. For example, voice-based wakeup is used, and the user may enter a preset wakeup instruction by using voice. For another example, wakeup is performed by using a fixed button on the detection device 100. Optionally, the fixed button may be a physical button or a virtual button.

Optionally, the detection device 100 may further include a pressure sensor, and the pressure sensor is configured to measure a body weight. When the detection device 100 is woken up by a body weight, the pressure sensor may detect a body weight of a user, and when the body weight of the user is detected, the detection device 100 is started to perform subsequent body composition detection.

When the detection device 100 includes a plurality of detectors, optionally, one pressure sensor may be disposed in each detector, and the pressure sensor is connected to the processor; or pressure sensors may be disposed in only some detectors, for example, a pressure sensor is disposed in only one of the detectors.

Optionally, the detection device 100 may further include a display 3. The display 3 is connected to and communicates with the processor. The display 3 may be configured to display a body composition measurement result, and may further display a body weight of a user and some prompt information. A size and a position of the display 3 may be shown in FIG. 1. Certainly, the size and the position of the display 3 are not limited to those shown in FIG. 1.

Optionally, the detection device 100 may further include a microphone and/or a loudspeaker, and the microphone and the loudspeaker are connected to and communicate with the processor. The microphone may be configured to detect a sound signal. For example, when the detection device 100 is woken up by voice, the microphone collects a sound signal of a user, and sends the collected sound signal to the processor for processing. After recognizing the sound signal, the processor determines whether to wake up the detection device 100. The loudspeaker is configured to play a sound signal, for example, may play a body composition detection result by using voice, or may play some prompt information.

Optionally, when the measured humidity value is greater than or equal to the preset humidity threshold, the processor is further configured to output first prompt information, where the first prompt information is used to prompt whether to perform dehumidification; and when a dehumidification confirmation instruction entered by the user based on the first prompt information is received, perform dehumidification by using the dehumidification assembly.

The processor may output the first prompt information in the following several manners.

Manner 1: When the detection device 100 includes the display, the detection device 100 sends the first prompt information to the display for displaying. The user may choose, by using a virtual button or a physical button on the display, whether to perform dehumidification; or the user enters a dehumidification instruction by using voice. The detection device 100 may collect, by using the microphone, a sound signal input by the user, and the processor recognizes the sound signal to obtain the dehumidification instruction. The dehumidification instruction is a dehumidification confirmation instruction or a dehumidification denial instruction. The dehumidification confirmation instruction is used to instruct the detection device 100 to perform dehumidification, and the dehumidification denial instruction is used to instruct the detection device 100 not to perform dehumidification.

Manner 2: When the detection device 100 includes the microphone and the loudspeaker, the detection device 100 may play the first prompt information by using the loudspeaker through voice. The user may enter a dehumidification instruction by using voice based on the first prompt information. The microphone collects a sound signal input by the user. The processor recognizes the sound signal to obtain the dehumidification instruction.

Manner 3: The detection device 100 sends the first prompt information to the terminal device. The terminal device displays the first prompt information by using the display or plays the first prompt information by using the loudspeaker. The user chooses, based on the first prompt information, whether to perform dehumidification. The terminal device sends a dehumidification instruction entered by the user to the detection device.

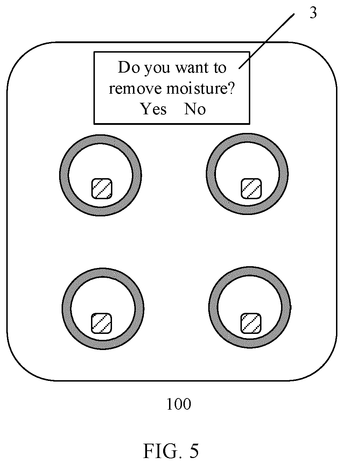

In a scenario, a user uses the detection device 100 to measure a body composition after exercise. After the user steps on the detection device 100, the detection device 100 is woken up, and the display of the detection device 100 is lit. In this case, the first prompt information is displayed on the display of the detection device 100. FIG. 5 is a schematic diagram of a display interface of the detection device. As shown in FIG. 5, for example, the first prompt information is as follows: "Do you want to remove moisture?" The user selects "Yes" or "No". When the user selects "Yes", the body composition detection device starts dehumidification. When the user selects "No", the body composition detection device ends body composition measurement, or skips a dehumidification process and directly measures body impedance.

When the display of the detection device 100 is a touchscreen, the user may select "Yes" or "No" by using a touch operation, or enter "Yes" or "No" by using voice. When the display of the detection device 100 is not a touchscreen, a physical button is disposed in the detection device 100, and the user selects "Yes" or "No" by operating the physical button, or enters "Yes" or "No" by using voice.

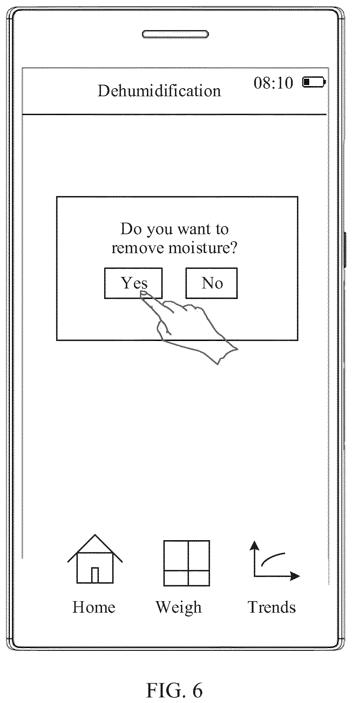

In another scenario, after exercise, a user uses the detection device 100 and a terminal device that controls the detection device 100 to perform body composition measurement. As shown in FIG. 3, the user starts the body fat scale app and taps the "Weigh" control to enter the interface shown in FIG. 3(b). After stepping on the detection device 100, the user taps a thumbnail of the electronic scale to trigger measurement, and an interface shown in FIG. 6 is displayed. As shown in FIG. 6, the interface displays the first prompt information and selection controls. For example, the first prompt information is as follows: "Do you want to remove moisture?" The selection controls include two controls: "Yes" and "No". After the user selects the "Yes" control, dehumidification starts. After the dehumidification ends, body composition measurement is performed, and a measurement result is displayed on the interface shown in FIG. 3(c).

It can be understood that FIG. 5 and FIG. 6 are merely schematic diagrams, and the first prompt information and the selection controls may alternatively be represented in other forms.

When the detection device 100 receives the dehumidification denial instruction entered by the user based on the first prompt information, optionally, the processor may further output second prompt information, where the second prompt information is used to prompt the user to perform detection after removing moisture. For example, the second prompt information is displayed on the display of the detection device 100, or the second prompt information is displayed on a display interface of the terminal device. For example, the second prompt information is as follows: "To ensure accuracy, please dry your hands and feet and perform measurement again."

Optionally, after dehumidification is completed, third prompt information is output, where the third prompt information is used to prompt a start of the body impedance measurement. For example, the third prompt information is as follows: "Moisture on the body surface is removed, and body impedance measurement is to start." In this way, the user can learn of measurement progress, thereby improving user experience. For example, after dehumidification is completed, the detection device 100 displays, on the display, the third prompt information: "Moisture on the body surface is removed, and body impedance measurement is to start." In a measurement process, the third prompt information is always displayed on the display. After the measurement is completed, the third prompt information is no longer displayed, and content displayed on the display changes to a body composition measurement result. Optionally, the third prompt information may alternatively disappear after being displayed for a preset time. For example, the third prompt information disappears after being displayed for 1 second or 2 seconds. In this case, no content may be displayed on the display, or notification information of "measuring..." may be displayed. The notification information may be displayed in a text form, an animation form, or another form.

Alternatively, after dehumidification is completed, the detection device 100 notifies the terminal device that dehumidification is completed, the terminal device displays the third prompt information on the display interface, and body composition measurement is performed. In a measurement process, the terminal device may always display the third prompt information. After the measurement is completed, the third prompt information is no longer displayed, and the page jumps to a body composition measurement result display interface. Optionally, the third prompt information may alternatively disappear after being displayed for a preset time. In this case, no content may be displayed on the interface of the terminal device, or notification information of "measuring..." may be displayed. The notification information may be displayed in a text form, an animation form, or another form.

The detection device 100 shown in FIG. 1 includes four detectors. In another embodiment of this application, the detection device may alternatively include more or fewer detectors. For example, the detection device 100 includes one detector, two detectors, six detectors, or eight detectors. In addition, a shape of the detector is not limited to a circular shape shown in FIG. 1, and another shape may alternatively be used. For example, the detector may alternatively be in an elliptic shape, a square shape, a rectangular shape, or another irregular shape. This is not limited in this embodiment.

It should be noted that, in this embodiment of this application, the detection device 100 may include one processor (or support layer), and a plurality of detectors share one processor, or one processor may be disposed for each detector, processors of the detectors can communicate with each other, and one of the processors may be specified to interact with the terminal device or another device.

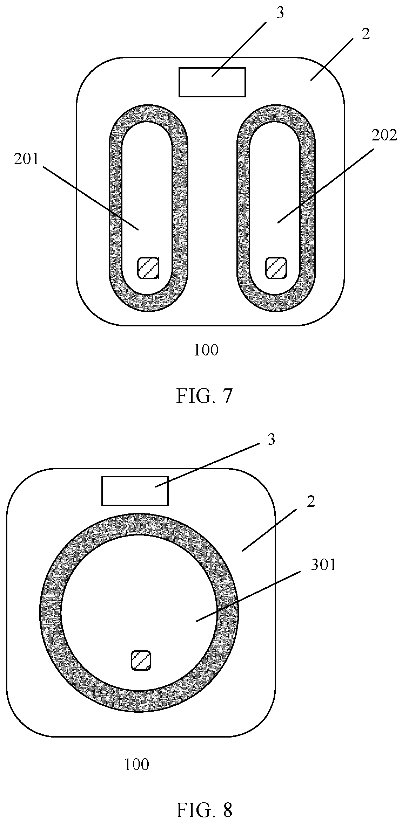

FIG. 7 is a schematic diagram of another external structure of a body composition detection device. As shown in FIG. 7, compared with the detection device shown in FIG. 1, the detection device includes two detectors: a detector 201 and a detector 202, and the detector 201 and the detector 202 are in an approximately elliptic shape. Certainly, shapes of the detector 201 and the detector 202 are not limited to that shown in FIG. 7. An area of the detector shown in FIG. 2 is larger than that of the detector shown in FIG. 1, to cover feet of a human body, and fully dehumidify the feet of the human body.

When the detection device 100 shown in FIG. 7 is used for measurement, both feet of a human body step on the detectors. For example, a left foot of the human body steps on the detector 201 and the detector 202. When the current- and voltage-based impedance measurement manner is used, a current flows into the human body from the detector 201, passes through the human body, and flows out of the human body from the detector 202. Electrodes of two impedance measurement assemblies of the detector 201 and the detector 202 are configured to measure a voltage. In this manner, a current loop is reused as a voltage loop. The impedance measurement assembly calculates body impedance based on the current and the measured voltage.

FIG. 8 is a schematic diagram of still another external structure of a body composition detection device. As shown in FIG. 8, compared with the detection device shown in FIG. 1, the detection device includes one detector 301, and the detector 301 is in a circular shape. Certainly, a shape of the detector 301 is not limited to that shown in FIG. 3, and the detector 301 may alternatively be in an elliptic shape or a square shape. An area of the detector 301 covers feet of a human body.

The current- and voltage-based impedance measurement manner may be used for the body composition detection device shown in FIG. 1 and FIG. 8. In the current- and voltage-based impedance measurement manner, a current loop and a voltage loop need to be formed through a human body. Certainly, impedance measurement may alternatively be performed in another manner, and no current loop or voltage loop needs to be formed. In the body composition detection device shown in FIG. 4, there is only one detector, and no current loop or voltage loop can be formed. Therefore, a non-current- or voltage-based impedance measurement manner may be used.

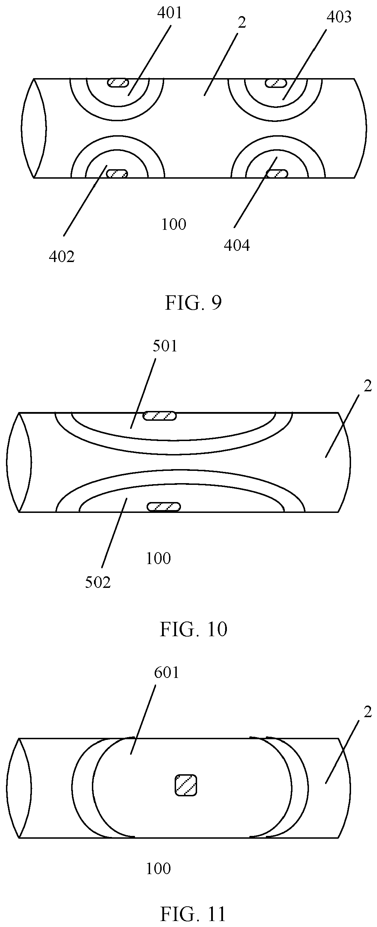

The detection device 100 shown in FIG. 1, FIG. 7, and FIG. 8 is a foot detection device. When the detection device is a hand detection device or includes a hand detection device, a structure shown in FIG. 9 may be used for the hand detection device. As shown in FIG. 9, the hand detection device may be in a cylindrical shape. During measurement, a user may hold the hand detection device by hand, to trigger the hand detection device to start measurement. The hand detection device includes a housing 2 and four detectors: a detector 401, a detector 402, a detector 403, and a detector 404.

A shape of the hand detection device is not limited in this embodiment. The hand detection device includes a detector and a housing. The hand detection device may include one or more detectors. For a structure of the detector of the hand detection device, refer to the structure of the detector of the foot detection device. Details are not described herein again.

FIG. 10 is a schematic diagram of another external structure of a hand detection device. As shown in FIG. 10, the hand detection device includes a housing 2 and two detectors: a detector 501 and a detector 502. An outer surface of each detector may circle around the hand detection device in a cylindrical shape.

FIG. 11 is a schematic diagram of still another external structure of a hand detection device. As shown in FIG. 11, the hand detection device includes a housing 2 and one detector: a detector 601. An outer surface of the detector 601 may circle around the hand detection device in a cylindrical shape.

Optionally, the hand detection device may further include one or more of the following components: a display, a microphone, or a loudspeaker. A wakeup manner of the hand detection device is the foregoing body weight-based wakeup, voice-based wakeup, or button-based wakeup. Details are not described herein again.

When the detection device includes a foot detection device and a hand detection device, a user may perform measurement by using both the foot detection device and the hand detection device. The hand detection device and the foot detection device may be connected and perform communication in a wired or wireless manner. When connection is performed in the wired manner, a connection wire may be separately connected to the hand detection device and the foot detection device through USB ports. The wireless manner may be Bluetooth connection or connection by using another short-range communication technology. During use, a user steps on the foot detection device by foot and holds the hand detection device by hand, to measure body impedance.

The hand detection device and the foot detection device may perform communication in the wired or wireless manner, to exchange measurement data. Usually, the foot detection device may be configured to detect a body weight and body impedance, while the hand detection device may be configured to detect only body impedance. The hand detection device may send detected body impedance to the foot detection device in a wired or wireless manner. The foot detection device processes the body weight, the body impedance measured by the foot detection device, and the body impedance measured by the hand detection device, to obtain a body composition.

Optionally, the foot detection device may alternatively send the body weight, the body impedance measured by the foot detection device, and the body impedance measured by the hand detection device to a mobile phone or another electronic device that is connected to the foot detection device. A mobile phone is used as an example. After receiving the body weight, the body impedance measured by the foot detection device, and the body impedance measured by the hand detection device, the mobile phone processes the body weight, the body impedance measured by the foot detection device, and the body impedance measured by the hand detection device, to obtain a body composition.

The body composition detection device provided in embodiments of this application includes the dehumidification assembly. The dehumidification assembly dehumidifies a body surface in contact with the impedance measurement assembly before the impedance measurement assembly measures body impedance. After moisture on the body surface is removed, the body impedance measurement assembly measures the body impedance. In this way, accuracy of the body impedance measurement is improved, and further, accuracy for detecting a body composition by using the body impedance can be improved.

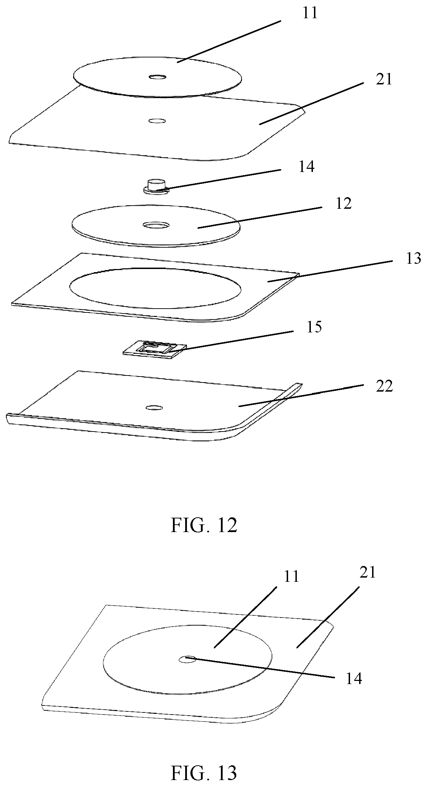

Embodiment 1 of this application provides a body composition detection device that performs dehumidification based on heating, and a dehumidification assembly is an electric heating element. FIG. 12 is a schematic exploded view of a detector of a body composition detection device that performs dehumidification based on heating. FIG. 13 is a schematic diagram of an appearance of the detector of the body composition detection device shown in FIG. 12. As shown in FIG. 12 and FIG. 13, the detector includes an impedance measurement assembly 11, an electric heating element 12, a support layer 13, a humidity measurement assembly 14, and a pressure sensor 15. In this embodiment, the housing 2 of the detection device 100 includes an upper housing 21 and a lower housing 22.

The impedance measurement assembly 11, the upper housing 21, the electric heating element 12, and the support layer 13 are stacked from top to bottom. A through hole is provided on each of the impedance measurement assembly 11, the upper housing 21, and the electric heating element 12. The through holes provided on the impedance measurement assembly 11, the upper housing 21, and the electric heating element 12 have a same size, and centers of the through holes are aligned. The humidity measurement assembly 14 is mounted in the through hole. A lower end of the humidity measurement assembly 14 is electrically connected to the support layer 13. An upper end of the humidity measurement assembly 14 sequentially passes through the electric heating element 12, the upper housing 21, and the impedance measurement assembly 11 from bottom to top.

Optionally, the through hole is provided at center positions on the impedance measurement assembly 11 and the electric heating element 12. It can be understood that the through hole may alternatively be provided at other positions on the impedance measurement assembly 11 and the electric heating element 12. This is not limited in this embodiment.

The electric heating element 12 is heated when being energized, and heat is transferred to a body surface through the upper housing 21 and the impedance measurement assembly 11, to dehumidify the body surface.

During body composition measurement, both an upper surface of the humidity measurement assembly 14 and an upper surface of the impedance measurement assembly 11 need to be in contact with the body surface. Optionally, in this embodiment, the upper surface of the humidity measurement assembly 14 and the upper surface of the impedance measurement assembly 11 are arranged to be located on one plane, and the upper surface of the impedance measurement assembly 11 is flush with or higher than an upper surface of the upper housing 21. This can ensure that the body surface can be in contact with the upper surface of the humidity measurement assembly 14 and the upper surface of the impedance measurement assembly 11 during body composition measurement. Optionally, the upper surface of the humidity measurement assembly 14 is slightly higher than the upper surface of the impedance measurement assembly 11.

The support layer 13 includes a processor, a connection wire (not shown in the figure), and the like. In this embodiment, the support layer 13 further has a supporting and mounting function. As shown in FIG. 7, an upper surface of the support layer 13 has a circular groove, and the electric heating element 12 in a circular shape may be accommodated in the groove, to fix the electric heating element 12. An electrode of the electric heating element 12 is connected to the support layer 13 through the wire.

Optionally, the pressure sensor 15 is mounted below the support layer 13. For example, there is a protrusion at a center of a lower surface of the pressure sensor 15, and the protrusion passes through a hole in the lower housing 22. The pressure sensor 15 is in a compressed state during measurement. Therefore, when the pressure sensor 15 is used for measurement, the bottom of the pressure sensor 15 needs to be supported, and the top of the pressure sensor 15 needs to be not blocked. In an optional manner, when the detection device is not used for measurement, the bottom of the pressure sensor 15 may or may not be in contact with the ground; or when the detection device is used for measurement, the bottom of the pressure sensor 15 is in contact with the ground because the pressure sensor 15 is compressed, so that the pressure sensor 15 can measure a body weight.

Optionally, the impedance measurement assembly 11 and the electric heating element 12 have circular structures. Optionally, the impedance measurement assembly 11 and the electric heating element 12 have a same size. It can be understood that the impedance measurement assembly 11 and the electric heating element 12 may alternatively be in a shape other than the circular shape, and may alternatively have different sizes.

Optionally, the impedance measurement assembly 11 is an impedance measurement electrode, and the impedance measurement electrode is formed by coating the upper surface of the upper housing 21.

Optionally, the impedance measurement assembly 11 is an independent assembly, and the impedance measurement assembly 11 is disposed in a groove on the upper housing 21.

The body composition detection device in this embodiment includes the electric heating element 12 and the humidity measurement assembly 14. After the detection device is woken up, the humidity measurement assembly 14 starts to measure humidity on a body surface. When a measured humidity value does not meet a measurement condition requirement, the electric heating element 12 starts to dehumidify the body surface. When a measured humidity value meets the measurement condition requirement, the electric heating element 12 ends the dehumidification operation, and the body impedance measurement assembly 11 measures body impedance. The electric heating element 12 dehumidifies the body surface before the body impedance is measured, and the body impedance is measured only after moisture on the body surface is removed. In this way, accuracy of the body impedance measurement is improved, and further, accuracy for detecting a body composition by using the body impedance can be improved.

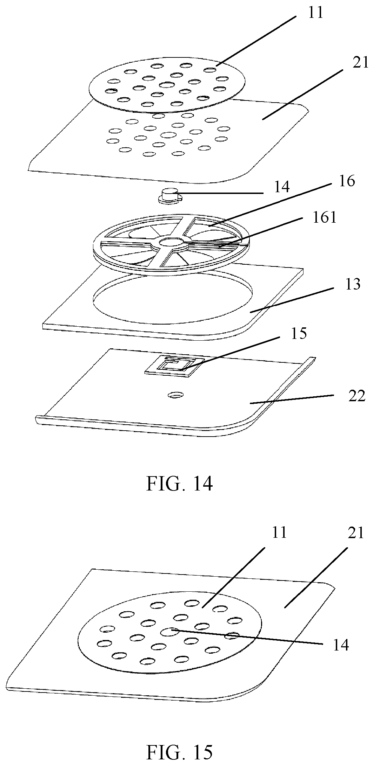

Embodiment 2 of this application provides a body composition detection device that performs dehumidification based on blowing, and a dehumidification assembly is a fan. FIG. 14 is a schematic exploded view of a detector of a body composition detection device that performs dehumidification based on blowing. FIG. 15 is a schematic diagram of an appearance of the detector of the body composition detection device shown in FIG. 14. As shown in FIG. 14 and FIG. 15, the detector 1 includes an impedance measurement assembly 11, a fan 16, a support layer 13, a humidity measurement assembly 14, and a pressure sensor 15. In this embodiment, the housing 2 of the detection device 100 includes an upper housing 21 and a lower housing 22.

The impedance measurement assembly 11, the upper housing 21, the fan 16, and the support layer 13 are stacked from top to bottom. A through hole is provided on each of the impedance measurement assembly 11 and the housing 21. The through holes provided on the impedance measurement assembly 11 and the upper housing 21 have a same size, and centers of the through holes are aligned. The humidity measurement assembly 14 is mounted in the through hole. An electrical connection wire of the humidity measurement assembly 14 and an electrical connection wire of the impedance measurement assembly 11 are connected to the processor of the support layer 13 through a wire duct 161 on an upper surface of the fan 16. A lower end of the humidity measurement assembly 14 is in contact with the fan 16. An upper end of the humidity measurement assembly 14 sequentially passes through the upper housing 21 and the impedance measurement assembly 11 from bottom to top. A plurality of air vents are provided at corresponding positions on the impedance measurement assembly 11 and the upper housing 21. Air blown out of the fan 16 is blown to a body surface through the air vents, to dehumidify the body surface.

In an optional embodiment, a through hole is provided on each of the impedance measurement assembly 11, the upper housing 21, and the fan 16, the humidity measurement assembly 14 is mounted in the through hole, a lower end of the humidity measurement assembly 14 is electrically connected to the support layer 13, and an upper end of the humidity measurement assembly 14 sequentially passes through the fan 16, the upper housing 21, and the impedance measurement assembly 11 from bottom to top.

Optionally, the through hole is provided at center positions on the impedance measurement assembly 11 and the fan 16. It can be understood that the through hole may alternatively be provided at other positions on the impedance measurement assembly 11 and the fan 16. This is not limited in this embodiment.

During body composition measurement, both an upper surface of the humidity measurement assembly 14 and an upper surface of the impedance measurement assembly 11 need to be in contact with the body surface. Therefore, in this embodiment, the upper surface of the humidity measurement assembly 14 and the upper surface of the impedance measurement assembly 11 are arranged to be located on one plane, and the upper surface of the impedance measurement assembly 11 is flush with or higher than an upper surface of the upper housing 21. This can ensure that the body surface can be in contact with the upper surface of the humidity measurement assembly 14 and the upper surface of the impedance measurement assembly 11 during body composition measurement. Optionally, the upper surface of the humidity measurement assembly 14 is slightly higher than the upper surface of the impedance measurement assembly 11.

The support layer 13 includes a processor, a connection wire (not shown in the figure), and the like. In this embodiment, the support layer 13 further has a supporting and mounting function. As shown in FIG. 9, an upper surface of the support layer 13 has a circular groove, and the fan 16 in a circular shape may be accommodated in the groove, to fix the fan 16. The fan 16 is connected to the support layer 13 through the wire.

Optionally, the pressure sensor 15 is mounted below the support layer 13. For example, there is a protrusion at a center of a lower surface of the pressure sensor 15, and the protrusion passes through a hole in the lower housing 22. For a mounting manner of the pressure sensor 15, refer to related descriptions in Embodiment 1. Details are not described herein again.

Optionally, the impedance measurement assembly 11 has a circular structure. It can be understood that the impedance measurement assembly may alternatively be in a shape other than the circular shape.

Optionally, the impedance measurement assembly 11 is an impedance measurement electrode, and the impedance measurement electrode is formed by coating the upper surface of the upper housing 21.

Optionally, the impedance measurement assembly 11 is an independent assembly, and the impedance measurement assembly 11 is disposed in a groove on the upper housing 21.

The body composition detection device in this embodiment includes the fan 16 and the humidity measurement assembly 14. After the detection device is woken up, the humidity measurement assembly 14 starts to measure humidity on a body surface. When a measured humidity value does not meet a measurement condition requirement, the fan 16 starts to dehumidify the body surface. In a dehumidification process of the fan 16, the humidity measurement assembly 14 continuously measures humidity on the body surface. When a measured humidity value meets the measurement condition requirement, the fan 16 stops dehumidifying the body surface, and the body impedance measurement assembly 11 starts to measure body impedance. The fan 16 dehumidifies the body surface before the body impedance is measured, and the body impedance is measured only after moisture on the body surface is removed. In this way, accuracy of the body impedance measurement is improved, and further, accuracy for detecting a body composition by using the body impedance can be improved.

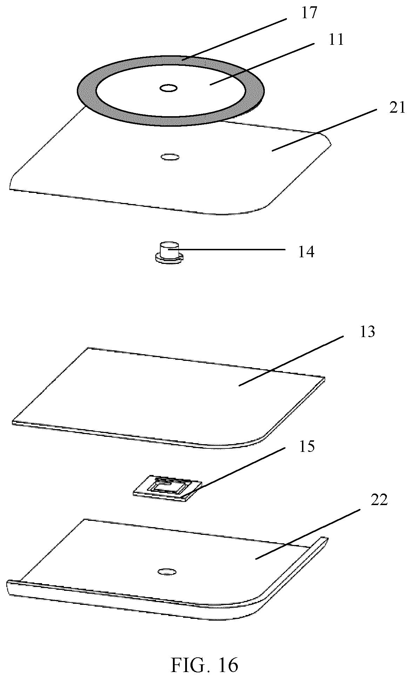

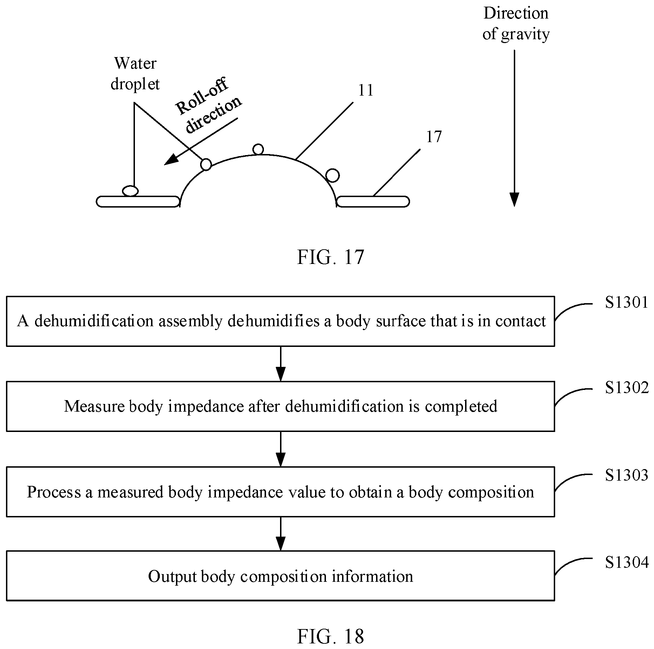

Embodiment 3 of this application provides a body composition detection device that performs dehumidification based on moisture absorption, and a dehumidification assembly is a drying ring. FIG. 16 is a schematic exploded view of a detector of a body composition detection device that performs dehumidification based on moisture absorption. FIG. 17 is a side view of an appearance of the detector of the body composition detection device shown in FIG. 16. As shown in FIG. 16 and FIG. 17, the detector 1 includes an impedance measurement assembly 11, a drying ring 17, a support layer 13, a humidity measurement assembly 14, and a pressure sensor 15. In this embodiment, the housing 2 of the detection device 100 includes an upper housing 21 and a lower housing 22.

The impedance measurement assembly 11 has an arc-shaped convex structure, and the drying ring 17 is arranged around the impedance measurement assembly 11. An upper surface of the drying ring 17 is lower than or flush with an outer edge of the impedance measurement assembly 11, so that a slope can be formed. In this way, water droplets on the body surface can fall from the top of the impedance measurement assembly 11 onto the drying ring 17, and the drying ring 17 absorbs moisture.

In this embodiment, the impedance measurement assembly 11, the upper housing 21, and the support layer 13 are stacked from top to bottom. A through hole is provided on each of the impedance measurement assembly 11 and the upper housing 21. The through holes provided on the impedance measurement assembly 11 and the upper housing 21 have a same size, and centers of the through holes are aligned. The humidity measurement assembly 14 is mounted in the through hole. A lower end of the humidity measurement assembly 14 is electrically connected to the support layer 13. An upper end of the humidity measurement assembly 14 sequentially passes through the upper housing 21 and the impedance measurement assembly 11 from bottom to top.

Optionally, a connection position between the upper surface of the drying ring 11 and the impedance measurement assembly 11 may have a smooth transition.

Optionally, the through hole is provided at a center position on the impedance measurement assembly 11. It can be understood that the through hole may alternatively be provided at another position on the impedance measurement assembly 11. This is not limited in this embodiment.

The impedance measurement assembly 11 is made of a super-hydrophobic conductive material, and has an arc-shaped convex structure. When a user performs measurement, water droplets on a body surface are under the action of gravity, where a direction of gravity is shown in FIG. 12. The water droplets fall from the top of a protrusion of the impedance measurement assembly 11 onto the drying ring 17 along various directions. The drying ring 17 is made of a superhydrophilic or super-absorbent material, and can rapidly absorb falling water droplets, to dehumidify the body surface.

During body composition measurement, both an upper surface of the humidity measurement assembly 14 and an upper surface of the impedance measurement assembly 11 need to be in contact with the body surface. Therefore, in this embodiment, the upper surface of the humidity measurement assembly 14 and the upper surface of the impedance measurement assembly 11 are arranged to be located on one plane, and the upper surface of the impedance measurement assembly 11 is flush with or higher than an upper surface of the upper housing 21. This can ensure that the body surface can be in contact with the upper surface of the humidity measurement assembly 14 and the upper surface of the impedance measurement assembly 11 during body composition measurement. Optionally, the upper surface of the humidity measurement assembly 14 is slightly higher than the upper surface of the impedance measurement assembly 11.

The support layer 13 includes a processor, a connection wire (not shown in the figure), and the like.

Optionally, the pressure sensor 15 is mounted below the support layer 13. For example, there is a protrusion at a center of a lower surface of the pressure sensor 15, and the protrusion passes through a hole in the lower housing 22. For a mounting manner of the pressure sensor 15, refer to related descriptions in Embodiment 1. Details are not described herein again.

Optionally, the impedance measurement assembly 11 has a circular structure, a shape of the drying ring 17 is the same as that of the impedance measurement assembly 11, and the drying ring 17 is disposed around the impedance measurement assembly 11. It can be understood that the impedance measurement assembly 11 and the drying ring 17 may alternatively be in a shape other than the circular shape.

In an optional embodiment, there may be a plurality of pairs of drying rings 17 and impedance measurement assemblies 11, the drying rings 17 and the impedance measurement assemblies 11 are disposed at intervals at a plurality of layers, the impedance measurement assembly 11 is at an innermost layer, and the drying ring 17 is at an outermost layer. Certainly, heights of the plurality of layers of drying rings 17 and impedance measurement assemblies 11 sequentially decrease from the inside out, thereby ensuring that water droplets fall from a higher position onto the drying ring.

The body composition detection device in this embodiment includes the drying ring 17 and the humidity measurement assembly 14. After the detection device is woken up, the drying ring 17 dehumidifies a body surface, and the humidity measurement assembly 14 starts to measure humidity on the body surface. When a measured humidity value does not meet a measurement condition requirement, the impedance measurement assembly 11 does not measure impedance. In a dehumidification process, the humidity measurement assembly 14 continuously measures humidity on the body surface. When a measured humidity value meets the measurement condition requirement, the impedance measurement assembly 11 starts to measure impedance. The drying ring 17 dehumidifies the body surface, and the body impedance is measured only after moisture on the body surface is removed. In this way, accuracy of the body impedance measurement is improved, and further, accuracy for detecting a body composition by using the body impedance can be improved.

In the body composition detection devices provided in Embodiment 1 to Embodiment 3, the support layer (or the processor) is described as a part of the detector. It can be understood that the support layer may not belong to a detector, but is a component shared by a plurality of detectors.

FIG. 18 is a flowchart of a body composition detection method according to Embodiment 4 of this application. The method provided in this embodiment may be performed by the body composition detection device in any one of the foregoing embodiments. As shown in FIG. 18, the method provided in this embodiment includes the following steps.

S1301: A dehumidification assembly dehumidifies a body surface in contact with the body composition detection device.

After the body composition detection device is woken up, the dehumidification assembly performs dehumidification. The body composition detection device may be woken up in one or more of the following wakeup manners: body weight-based wakeup, voice-based wakeup, or button-based wakeup.

The body weight-based wakeup means: After a human body is in contact with the body composition detection device, the body composition detection device detects a weight of a user, and the detected weight is greater than a specified threshold, so that the body composition detection device is woken up. For example, a foot detection device is woken up after the user stands on the foot detection device, and a hand detection device is woken up after the user holds the hand detection device by hand.

The voice-based wakeup means: A user enters a preset wakeup instruction by using voice, and after the body composition detection device recognizes the preset wakeup instruction, the body composition detection device is woken up.

The button-based wakeup means: A user wakes up the body composition detection device by pressing or touching a fixed button on the body composition detection device. Optionally, the fixed button may be a physical button or a virtual button.

In an optional implementation, after the body composition detection device is woken up, the dehumidification assembly immediately dehumidifies the body surface in contact with the body composition detection device. After the dehumidification ends, body impedance measurement starts. The dehumidification assembly may perform dehumidification based on a preset dehumidification time. After the dehumidification time expires, the dehumidification ends. By default, it is considered that the body surface is dry and body impedance measurement can be performed. The dehumidification time may be a system default value, or may be flexibly set by a user. For example, the dehumidification time may be 1 minute, 2 minutes, 3 minutes, or 5 minutes.

In another optional implementation, the dehumidification assembly starts dehumidification under a specific trigger condition. For example, before the dehumidification assembly dehumidifies the body surface in contact with the body composition detection device, humidity on the body surface is measured, and whether a measured humidity value of the body surface meets a measurement condition requirement is determined. When the humidity value of the body surface does not meet the measurement condition requirement, the dehumidification assembly dehumidifies the body surface; or when the humidity value of the body surface meets the measurement condition requirement, the body surface does not need to be dehumidified, and body impedance is directly measured.

Alternatively, the body composition detection device sends the measured humidity value of the body surface to a terminal device, and the terminal device determines whether the humidity value of the body surface meets the measurement condition requirement. When the humidity value of the body surface does not meet the measurement condition requirement, the terminal device indicates the body composition detection device to perform dehumidification; or when the humidity value of the body surface meets the measurement condition requirement, the terminal device indicates the body composition detection device to start body impedance measurement.

Optionally, the terminal device further sends a dehumidification time to the body composition, and the body composition detection device starts to measure body impedance after the dehumidification time expires. Alternatively, the terminal device does not send a dehumidification time to the body composition detection device, but sends second indication information to the body composition detection device after the dehumidification time expires or when the humidity value of the body surface meets the measurement condition requirement, where the second indication information is used to indicate to stop dehumidification and start body impedance measurement.

Optionally, the measurement condition requirement is as follows: Dehumidification needs to be performed when a humidity value is greater than or equal to a preset humidity threshold. For example, after the body composition detection device is woken up, the body composition detection device starts to measure the humidity on the body surface; and when the measured humidity value is greater than or equal to the preset humidity threshold, the dehumidification assembly starts to dehumidify the body surface; or when the measured humidity value is less than the preset humidity threshold, the body impedance is measured.

When the measured humidity value is greater than or equal to the preset humidity threshold, the dehumidification assembly may perform dehumidification based on a preset dehumidification time. After the dehumidification time expires, the dehumidification operation ends. Alternatively, the body composition detection device determines a dehumidification time based on the measured humidity value, and controls the dehumidification assembly to perform dehumidification based on the determined dehumidification time. Alternatively, in a dehumidification process of the dehumidification assembly, the body composition detection device continuously measures the humidity on the body surface, and when a measured humidity value meets the measurement condition requirement of an impedance measurement assembly, the dehumidification assembly is controlled to end the dehumidification operation.

Optionally, when the humidity value of the body surface does not meet the measurement condition requirement, first prompt information is output, where the first prompt information is used to prompt whether to perform dehumidification. A user enters a dehumidification confirmation instruction or a dehumidification denial instruction based on the first prompt information. When the dehumidification confirmation instruction entered by the user based on the first prompt information is received, the dehumidification assembly dehumidifies the body surface. For example, the first prompt information is as follows: "Do you want to remove moisture?" The user selects "Yes" or "No". When the user selects "Yes", the body composition detection device starts dehumidification. When the user selects "No", the body composition detection device may directly measure the body impedance without dehumidification.

Optionally, when the dehumidification denial instruction entered by the user based on the first prompt information is received, second prompt information is output, where the second prompt information is used to prompt the user to perform detection after removing moisture. For example, the second prompt information is as follows: "To ensure accuracy, please dry your hands and feet and perform measurement again."

Optionally, after outputting the second prompt information, the body composition detection device may end a measurement process, or may continue to measure body impedance, and obtain a body composition based on the body impedance. The user may learn, based on the second prompt information, that the measured body composition is inaccurate and is for reference only.

In still another optional manner, before the dehumidification assembly dehumidifies the body surface in contact with the body composition detection device, the dehumidification assembly receives first indication information sent by the terminal device, where the first indication information is used to indicate to perform dehumidification before the body impedance is measured, and the dehumidification assembly dehumidifies the body surface based on the first indication information. The terminal device is configured to control the body composition detection device.

Optionally, the body composition detection device further receives a dehumidification time sent by the terminal device, and when the dehumidification time expires, determines that dehumidification is completed. The first indication information and the dehumidification time may be sent by the terminal device to the body composition detection device by using one message, or may be sent to the body composition detection device by using two messages.

Optionally, the dehumidification assembly performs dehumidification in one or more of the following manners: heating, blowing, or moisture absorption. When dehumidification is performed through heating, the dehumidification assembly may be an electric heating element, and the electric heating element is heated after being energized. When dehumidification is performed through blowing, the dehumidification assembly may be a fan, and dehumidification is performed by accelerating air circulation through blowing. When dehumidification is performed through moisture absorption, the dehumidification assembly may be a drying ring with good moisture absorption performance, and the drying ring absorbs moisture after getting in contact with the body surface.

S1302: Measure body impedance after dehumidification is completed.

After the body surface is dehumidified, the body surface is dry, and body impedance may be measured. The measured body impedance is accurate.

Optionally, after dehumidification is completed, the body composition detection device may further output third prompt information, where the third prompt information is used to prompt a start of the body impedance measurement. For example, the third prompt information is as follows: "Body impedance measurement is to start", "Moisture on hands or feet is removed", or "Moisture on hands or feet is removed, and body impedance measurement is to start".

In another possible embodiment of this application, after dehumidification is completed, no third prompt information is output, and the body composition detection device starts body impedance measurement by default.

S1303: Process a measured body impedance value to obtain a body composition.

An existing method may be used to convert the body impedance into the body composition. This is not limited in this embodiment of this application.

S1304: Output body composition information.

The body composition detection device may output the body composition in one or more of the following manners: (1) When the body composition detection device includes a display, the body composition is sent to the display for displaying. (2) The body composition is played by using voice. (3) The body composition is sent to the terminal device, and the terminal device displays the body composition and/or plays the body composition by using voice.

Optionally, in another embodiment of this application, after measuring the body impedance, the body composition detection device does not process the body impedance, but sends the body impedance to the terminal device, and the terminal device processes the body impedance to obtain a body composition.

In the method of this embodiment, the body surface is dehumidified before the body impedance is measured, and the body impedance measurement starts only after moisture on the body surface is removed. In this way, accuracy of the body impedance measurement is improved, and further, accuracy for detecting the body composition by using the body impedance can be improved.

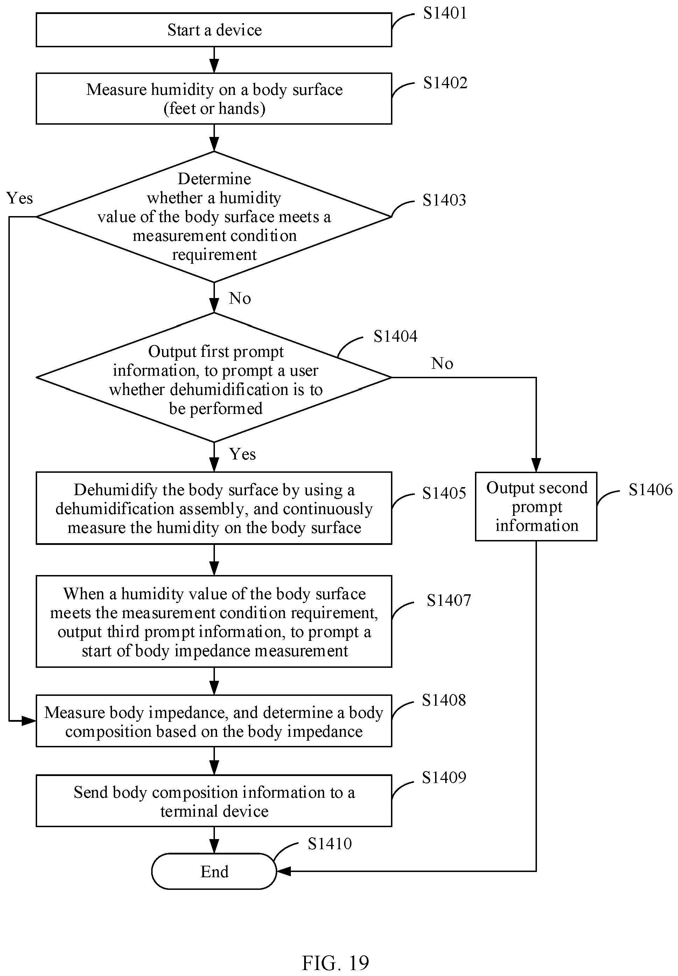

Based on the method in Embodiment 4, FIG. 19 is a flowchart of a body composition detection method according to Embodiment 5 of this application which is not a claimed embodiment. The method provided in this embodiment may be performed by the body composition detection device in any one of the foregoing embodiments. As shown in FIG. 19, the method provided in this embodiment includes the following steps.

S1401: Start a body composition detection device.

S1402: Measure humidity on a body surface (feet or hands).

S1403: Determine whether a humidity value of the body surface meets a measurement condition requirement.

If the humidity value of the body surface meets the measurement condition requirement, step S1408 is performed; or if the humidity value of the body surface does not meet the measurement condition requirement, step S 1404 is performed.

S1404: Output first prompt information, to prompt a user whether dehumidification is to be performed.

When the user chooses to perform dehumidification, step S1405 is performed, and step S1407 is performed after step S1405. When the user chooses not to perform dehumidification, step S1406 is performed, and the process ends after step S1406.

S1405: Dehumidify the body surface by using a dehumidification assembly, and continuously measure the humidity on the body surface.

S1406: Output second prompt information.

The second prompt information is used to prompt the user to perform detection after removing moisture.

S1407: When a humidity value of the body surface meets the measurement condition requirement, output third prompt information, to prompt a start of body impedance measurement.

S1408: Measure body impedance, and determine a body composition based on the body impedance.

S1409: Send body composition information to a terminal device.

S1410: End.

For a specific implementation of this embodiment, refer to related descriptions in Embodiment 4. Details are not described herein again.

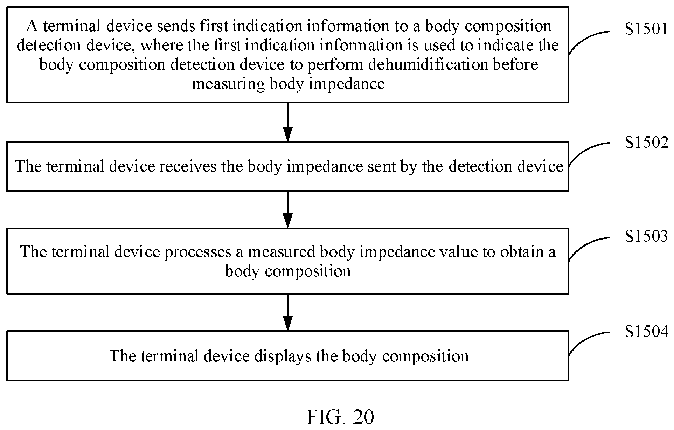

FIG. 20 is a flowchart of a body composition detection method according to Embodiment 5 of this application which is not a claimed embodiment. The method provided in this embodiment may be performed by a terminal device. As shown in FIG. 20, the method provided in this embodiment includes the following steps.

S1501: The terminal device sends first indication information to a body composition detection device, where the first indication information is used to indicate the body composition detection device to perform dehumidification before measuring body impedance.

Optionally, before the terminal device sends the first indication information to the body composition detection device, the terminal device displays first prompt information, where the first prompt information is used to prompt whether to perform dehumidification. For a displaying form of the first prompt information, refer to FIG. 6. A user enters a dehumidification confirmation instruction or a dehumidification denial instruction based on the first prompt information. When the dehumidification confirmation instruction entered by the user based on the first prompt information is received, the terminal device sends the first indication information to the body composition detection device. When the dehumidification denial instruction entered by the user based on the first prompt information is received, second prompt information is displayed, where the second prompt information is used to prompt the user to perform detection after removing moisture.

In an implementation, after the user triggers measurement, the terminal device displays the first prompt information by default. In another implementation, before outputting the first prompt information, the terminal device receives a humidity value of a body surface that is sent by the detection device; and when the humidity value of the body surface does not meet a measurement condition requirement, the terminal device displays the first prompt information. After being woken up, the detection device starts to measure a humidity value of the body surface, and sends the measured humidity value to the terminal device.

In a dehumidification process of the detection device, the detection device may continuously or periodically detect a humidity value of the body surface, and send the humidity value of the body surface to the terminal device. When the terminal device determines that the humidity value of the body surface meets the measurement condition requirement, the terminal device sends second indication information to the detection device, to indicate to stop dehumidification and start body impedance measurement.

Optionally, in a dehumidification process of the detection device, the detection device does not detect a humidity value of the body surface, and the detection device ends dehumidification after a preset dehumidification time of a system, and measures body impedance. Alternatively, the dehumidification time is sent by the terminal device to the detection device. Alternatively, the terminal device sends the second indication information to the detection device based on an indication of the user or the preset dehumidification time.

S1502: The terminal device receives the body impedance sent by the detection device.

After performing dehumidification based on the first indication information, the detection device measures body impedance, and sends the measured body impedance to the terminal device.

Optionally, after the terminal device determines that the detection device completes dehumidification and before body impedance measurement starts, the terminal device displays third prompt information, where the third prompt information is used to prompt a start of the body impedance measurement.

S1503: The terminal device processes a measured body impedance value to obtain a body composition.

S1504: The terminal device displays the body composition.

In the several embodiments provided in this application, it should be understood that the disclosed system, apparatus, and method may be implemented in other manners. For example, the described apparatus embodiment is merely an example. For example, division into the units is merely logical function division and may be other division in actual implementation. For example, a plurality of units or components may be combined or integrated into another system, or some features may be ignored or not performed. In addition, the displayed or discussed mutual couplings or direct couplings or communication connections may be implemented by using some interfaces. The indirect couplings or communication connections between the apparatuses or units may be implemented in electronic, mechanical, or other forms.

## Claims

1. A body composition detection device (100), comprising a housing (2), and at least one detector (101, 102, 103, 104), wherein
the detector (101, 102, 103, 104) comprises an impedance measurement assembly (11) and a dehumidification assembly, both the impedance measurement assembly (11) and the dehumidification assembly are electrically connected to a processor, and the impedance measurement assembly (11), the dehumidification assembly, and the processor are all disposed inside the housing (2); and
the impedance measurement assembly (11) is embedded on an upper surface of the housing (2), an upper surface of the impedance measurement assembly (11) is flush with or higher than the upper surface of the housing (2), and the upper surface of the impedance measurement assembly (11) is configured to be in contact with a human body during measurement,
**characterized in that** the body composition detection device (100) further comprises a support layer (13) that comprises the processor,
and **in that** the detector (101, 102, 103, 104) further comprises a humidity measurement assembly (14); and
the humidity measurement assembly (14) is electrically connected to the processor, the humidity measurement assembly (14) is embedded in the impedance measurement assembly (11), and an upper surface of the humidity measurement assembly (14) is configured to be in contact with a human body during measurement.

2. The device (100) according to claim 1, wherein the dehumidification assembly is an electric heating element (12); and
the impedance measurement assembly (11), the housing (2), the electric heating element (12), and the support layer (13) are stacked from top to bottom, a through hole is provided on each of the impedance measurement assembly (11), the housing (2), and the electric heating element (12), the humidity measurement assembly (14) is mounted in the through hole, and a lower end of the humidity measurement assembly (14) is electrically connected to the support layer (13).

3. The device (100) according to claim 2, wherein the through hole is provided at centers of the impedance measurement assembly (11) and the electric heating element (12).

4. The device (100) according to claim 1, wherein the dehumidification assembly is a fan (16);
the impedance measurement assembly (11), the housing (2), the fan (16), and the support layer (13) are stacked from top to bottom, a through hole is provided on each of the impedance measurement assembly (11), the housing, and the fan (16), the humidity measurement assembly (14) is mounted in the through hole, and a lower end of the humidity measurement assembly (14) is electrically connected to the processor; and
a plurality of air vents are provided at corresponding positions on the impedance measurement assembly (11) and the housing.

5. The device (100) according to claim 1, wherein the dehumidification assembly is a fan (16);
the impedance measurement assembly (11), the housing (2), the fan (16), and the support layer (13) are stacked from top to bottom, a through hole is provided on each of the impedance measurement assembly (11) and the housing (2), the humidity measurement assembly (14) is mounted in the through hole, and an electrode of the humidity measurement assembly (14) and an electrode of the impedance measurement assembly (11) are connected to the processor through a wire duct (161) on an upper surface of the fan (16); and
a plurality of air vents are provided at corresponding positions on the impedance measurement assembly (11) and the housing (2).

6. The device (100) according to claim 4, wherein the through hole is provided at centers of the impedance measurement assembly (11), the housing (2), and a rotating shaft of the fan (16).

7. The device (100) according to claim 5, wherein the through hole is provided at centers of the impedance measurement assembly (11) and the housing (2).

8. The device (100) according to any of the previous claims,
wherein when a humidity value measured by the humidity measurement assembly (14) does not meet a measurement condition requirement of the impedance measurement assembly (11), the dehumidification assembly is configured to perform dehumidification.

## Patentansprüche

1. Vorrichtung (100) zum Erkennen einer Körperzusammensetzung, umfassend ein Gehäuse (2) und mindestens einen Detektor (101, 102, 103 104), wobei:
der Detektor (101, 102, 103 104) eine Anordnung (11) zur Impedanzmessung und eine Entfeuchtungsanordnung umfasst, wobei sowohl die Anordnung (11) zur Impedanzmessung als auch die Entfeuchtungsanordnung elektrisch mit einem Prozessor verbunden sind und die Anordnung (11) zur Impedanzmessung, die Entfeuchtungsanordnung und der Prozessor alle im Inneren des Gehäuses (2) angeordnet sind, und
die Anordnung (11) zur Impedanzmessung in eine Oberseite des Gehäuses (2) eingebettet ist, eine Oberseite der Anordnung (11) zur Impedanzmessung mit der Oberseite des Gehäuses (2) bündig ist oder höher als diese liegt und die Oberseite der Anordnung (11) zur Impedanzmessung dafür gestaltet ist, während einer Messung mit einem menschlichen Körper in Kontakt zu stehen,
**dadurch gekennzeichnet, dass** die Vorrichtung (100) zum Erkennen einer Körperzusammensetzung ferner eine Trägerschicht (13) umfasst, die den Prozessor umfasst, und dadurch, dass der Detektor (101, 102, 103 104) ferner eine Anordnung (14) zur Feuchtigkeitsmessung umfasst, und
die Anordnung (14) zur Feuchtigkeitsmessung elektrisch mit dem Prozessor verbunden ist, die Anordnung (14) zur Feuchtigkeitsmessung in die Anordnung (11) zur Impedanzmessung eingebettet ist und eine Oberseite der Anordnung (14) zur Feuchtigkeitsmessung dafür gestaltet ist, während einer Messung mit einem menschlichen Körper in Kontakt zu stehen.

2. Vorrichtung (100) nach Anspruch 1, wobei die Entfeuchtungsanordnung ein elektrisches Heizelement (12) ist und
die Anordnung (11) zur Impedanzmessung, das Gehäuse (2), das elektrische Heizelement (12) und die Trägerschicht (13) von oben nach unten gestapelt sind, in jeweils der Anordnung (11) zur Impedanzmessung, dem Gehäuse (2) und dem elektrischen Heizelement (12) eine Durchgangsöffnung bereitgestellt ist, die Anordnung (14) zur Feuchtigkeitsmessung in der Durchgangsöffnung montiert ist und ein unteres Ende der Anordnung (14) zur Feuchtigkeitsmessung elektrisch mit der Trägerschicht (13) verbunden ist.

3. Vorrichtung (100) nach Anspruch 2, wobei die Durchgangsöffnung jeweils in der Mitte der Anordnung (11) zur Impedanzmessung und des elektrischen Heizelements (12) bereitgestellt ist.

4. Vorrichtung (100) nach Anspruch 1, wobei die Entfeuchtungsanordnung ein Gebläse (16) ist,
die Anordnung (11) zur Impedanzmessung, das Gehäuse (2), das Gebläse (16) und die Trägerschicht von oben nach unten gestapelt sind, in jeweils der Anordnung (11) zur Impedanzmessung, dem Gehäuse und dem Gebläse (16) eine Durchgangsöffnung bereitgestellt ist, die Anordnung (14) zur Feuchtigkeitsmessung in der Durchgangsöffnung montiert ist und ein unteres Ende der Anordnung (14) zur Feuchtigkeitsmessung elektrisch mit dem Prozessor verbunden ist und
an entsprechenden Positionen an der Anordnung (11) zur Impedanzmessung und dem Gehäuse mehrere Lüftungsöffnungen bereitgestellt sind.

5. Vorrichtung (100) nach Anspruch 1, wobei die Entfeuchtungsanordnung ein Gebläse (16) ist,
die Anordnung (11) zur Impedanzmessung, das Gehäuse (2), das Gebläse (16) und die Trägerschicht (13) von oben nach unten gestapelt sind, in jeweils der Anordnung (11) zur Impedanzmessung und dem Gehäuse (2) eine Durchgangsöffnung bereitgestellt ist, die Anordnung (14) zur Feuchtigkeitsmessung in der Durchgangsöffnung montiert ist und eine Elektrode der Anordnung (14) zur Feuchtigkeitsmessung und eine Elektrode der Anordnung (11) zur Impedanzmessung durch einen Kabelkanal (161) an einer Oberseite des Gebläses (16) mit dem Prozessor verbunden sind und
an entsprechenden Positionen an der Anordnung (11) zur Impedanzmessung und dem Gehäuse (2) mehrere Lüftungsöffnungen bereitgestellt sind.

6. Vorrichtung (100) nach Anspruch 4, wobei die Durchgangsöffnung jeweils in der Mitte der Anordnung (11) zur Impedanzmessung, des Gehäuses (2) und einer Drehwelle des Gebläses (16) bereitgestellt ist.

7. Vorrichtung (100) nach Anspruch 5, wobei die Durchgangsöffnung jeweils in der Mitte der Anordnung (11) zur Impedanzmessung und des Gehäuses (2) bereitgestellt ist.

8. Vorrichtung (100) nach einem der vorhergehenden Ansprüche,
wobei die Entfeuchtungsanordnung dafür gestaltet ist, eine Entfeuchtung durchzuführen, wenn ein Feuchtigkeitswert, der durch die Anordnung (14) zur Feuchtigkeitsmessung gemessen wird, eine Anforderung an die Messbedingungen der Anordnung (11) zur Impedanzmessung nicht erfüllt.

## Revendications

1. Dispositif de détection de composition corporelle (100), comprenant un boîtier (2), et au moins un détecteur (101, 102, 103, 104),
le détecteur (101, 102, 103, 104) comprenant un ensemble de mesure d'impédance (11) et un ensemble de déshumidification, l'ensemble de mesure d'impédance (11) et l'ensemble de déshumidification étant tous deux connectés électriquement à un processeur, et l'ensemble de mesure d'impédance (11), l'ensemble de déshumidification, et le processeur étant tous disposés à l'intérieur du boîtier (2) ; et
l'ensemble de mesure d'impédance (11) étant intégré sur une surface supérieure du boîtier (2), une surface supérieure de l'ensemble de mesure d'impédance (11) étant affleurante avec ou plus haute que la surface supérieure du boîtier (2), et la surface supérieure de l'ensemble de mesure d'impédance (11) étant configurée pour être en contact avec un corps humain pendant la mesure,
**caractérisé en ce que** le dispositif de détection de composition corporelle (100) comprend en outre une couche de support (13) qui comprend le processeur, et **en ce que** le détecteur (101, 102, 103, 104) comprend en outre un ensemble de mesure d'humidité (14), et
l'ensemble de mesure d'humidité (14) étant connecté électriquement au processeur, l'ensemble de mesure d'humidité (14) étant intégré dans l'ensemble de mesure d'impédance (11), et une surface supérieure de l'ensemble de mesure d'humidité (14) étant configurée pour être en contact avec un corps humain pendant la mesure.

2. Dispositif (100) selon la revendication 1, l'ensemble de déshumidification étant un élément chauffant électrique (12) ; et
l'ensemble de mesure d'impédance (11), le boîtier (2), l'élément chauffant électrique (12), et la couche de support (13) étant empilés de haut en bas, un trou traversant étant prévu sur chacun de l'ensemble de mesure d'impédance (11), du boîtier (2) et de l'élément chauffant électrique (12), l'ensemble de mesure d'humidité (14) étant monté dans le trou traversant, et une extrémité inférieure de l'ensemble de mesure d'humidité (14) étant connectée électriquement à la couche de support (13).

3. Dispositif (100) selon la revendication 2, le trou traversant étant prévu aux centres de l'ensemble de mesure d'impédance (11) et de l'élément chauffant électrique (12).

4. Dispositif (100) selon la revendication 1, l'ensemble de déshumidification étant un ventilateur (16) ;
l'ensemble de mesure d'impédance (11), le boîtier (2), le ventilateur (16), et la couche de support (13) étant empilés de haut en bas, un trou traversant étant prévu sur chacun de l'ensemble de mesure d'impédance (11), du boîtier et du ventilateur (16), l'ensemble de mesure d'humidité (14) étant monté dans le trou traversant, et une extrémité inférieure de l'ensemble de mesure d'humidité (14) étant connectée électriquement au processeur ; et
une pluralité d'évents d'air étant fournis à des positions correspondantes sur l'ensemble de mesure d'impédance (11) et le boîtier.

5. Dispositif (100) selon la revendication 1, l'ensemble de déshumidification étant un ventilateur (16) ;
l'ensemble de mesure d'impédance (11), le boîtier (2), le ventilateur (16) et la couche de support (13) étant empilés de haut en bas, un trou traversant étant prévu sur chacun de l'ensemble de mesure d'impédance (11) et du boîtier (2), l'ensemble de mesure d'humidité (14) étant monté dans le trou traversant, et une électrode de l'ensemble de mesure d'humidité (14) et une électrode de l'ensemble de mesure d'impédance (11) étant connectées au processeur par l'intermédiaire d'un conduit de câble (161) sur une surface supérieure du ventilateur (16) ; et
une pluralité d'évents d'air étant fournis à des positions correspondantes sur l'ensemble de mesure d'impédance (11) et le boîtier (2).

6. Dispositif (100) selon la revendication 4, le trou traversant étant prévu aux centres de l'ensemble de mesure d'impédance (11), du boîtier (2) et d'un arbre rotatif du ventilateur (16).

7. Dispositif (100) selon la revendication 5, le trou traversant étant prévu aux centres de l'ensemble de mesure d'impédance (11) et du boîtier (2).

8. Dispositif (100) selon l'une quelconque des revendications précédentes,
lorsqu'une valeur d'humidité mesurée par l'ensemble de mesure d'humidité (14) ne satisfait pas à une exigence de condition de mesure de l'ensemble de mesure d'impédance (11), l'ensemble de déshumidification étant configuré pour effectuer une déshumidification.
